(19) Europäisches Patentamt
European Patent Office
Office européen des brevets

(11) Veröffentlichungsnummer: **0 216 204**
**B1**

(12) # EUROPÄISCHE PATENTSCHRIFT

(45) Veröffentlichungstag der Patentschrift: 12.04.89

(51) Int. Cl.⁴: C 07 C 25/22, C 07 C 17/12

(21) Anmeldenummer: 86112090.5

(22) Anmeldetag: 02.09.86

ERRATUM

| DIE TEXTSTELLE: TEXT PUBLISHED: LE PASSAGE SUIVANT: | (SEITE, SPALTE, ZEILE) (PAGE, COLUMN, LINE) (PAGE, COLONNE, LIGNE) | | | LAUTET BERICHTIGT: SHOULD READ: DEVRAIT ETRE LU: |
|---|---|---|---|---|
| Man erählt 557 Teile | 5 | 7 | 33 | Man erhält 557 Teile |
| 2 Stunden bei 40° - 50°C Teile | 5 | 8 | 34/35 | 2 Stunden bei 40° - 50°C 380 Teile |
| le pyrène en suspension du pyrène á l'eau | 6 | 10 | 39 | le pyrène en suspension aqueuse très concentrée, selon un rapport ponderal du pyrène á l'eau |
| dans l'intervalle de ph | 6 | 10 | 52 | dans l'intervalle de pH |

Tag der Entscheidung über die Berichtigung / Date of decision on rectification / Date de décision portant sur modification: 14.06.89

Ausgabe- und Veröffentlichungstag / Issue and publication date / Date d'edition et de publication: 11.10.89

Patbl. Nr / EPB no / Bull. no: 89/41

(19) Europäisches Patentamt
European Patent Office
Office européen des brevets

(11) Veröffentlichungsnummer: **0 216 204**
**B1**

(12) # EUROPÄISCHE PATENTSCHRIFT

(45) Veröffentlichungstag der Patentschrift: **12.04.89**

(51) Int. Cl.⁴: **C 07 C 25/22**, C 07 C 17/12

(21) Anmeldenummer: **86112090.5**

(22) Anmeldetag: **02.09.86**

(54) **Verfahren zur Herstellung von 1,3,6,8-Tetrabrompyren.**

(30) Priorität: **14.09.85 DE 3532882**

(43) Veröffentlichungstag der Anmeldung: **01.04.87 Patentblatt 87/14**

(45) Bekanntmachung des Hinweises auf die Patenterteilung: **12.04.89 Patentblatt 89/15**

(84) Benannte Vertragsstaaten: **CH DE FR GB IT LI**

(56) Entgegenhaltungen:
**BE-A- 565 995**
**DE-C- 937 646**

(73) Patentinhaber: **HOECHST AKTIENGESELLSCHAFT, Postfach 80 03 20, D-6230 Frankfurt am Main 80 (DE)**

(72) Erfinder: **Schiessler, Siegfried, Dr., Rother Weingartenweg 48, D-6232 Bad Soden am Taunus (DE)**
Erfinder: **Spietschka, Ernst, Dr., Am Rödchen 8, D-6270 Idstein/Taunus (DE)**

**EP 0 216 204 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

1,3,6,8-Tetrabrompyren der Formel (1) stellt ein wichtiges Zwischenprodukt bei der Herstellung der Naphthalin-1,4,5,8-tetracarbonsäure dar.

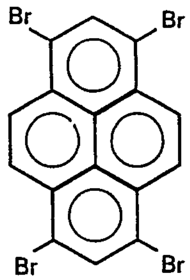

(1)

Bekanntermaßen [Annalen 531 (1937), 12] kann die Herstellung von 1,3,6,8-Tetrabrompyren durch Bromierung von Pyren in geeigneten inerten Lösemitteln, wie beispielsweise Nitrobenzol oder Trichlorbenzol, bei hoher Temperatur erfolgen. Doch ist dieses Verfahren nicht wirtschaftlich, da beim Arbeiten mit derartigen Lösemitteln nicht zuletzt aus physiologischen und ökologischen Gründen ein sehr hoher technischer Aufwand erforderlich ist.

Weiterhin ist aus DE-PS 937 646 bekannt, daß man Pyren in wäßrigem Medium zu 1,3,6,8-Tetrabrompyren bromieren kann und daß man dabei zur Reduzierung der Brommenge den bei der Bromierung entstehenden Bromwasserstoff durch ein Oxidationsmittel zu Brom reoxidieren kann. Dieses Verfahren ist zwar interessant, doch scheitert seine wirtschaftliche Realisierbarkeit daran, daß man das Pyren vor der Bromierung einer sehr aufwendigen Rollmahlung unterziehen muß. Außerdem erfordert dieses Verfahren den Einsatz von grenzflächenaktiven Verbindungen und von Schwefelsäure, wodurch bei der Isolierung des Tetrabrompyrens ökologisch belastete Filtrate auftreten. Außerdem spaltet das nach der DE-PS 937 646 bei der Filtration anfallende Tetrabrompyren trotz intensiven Waschens des Filterkuchens mit Wasser bei der Trocknung noch Bromwasserstoff ab, wodurch erhebliche Korrosionsprobleme bei den Trockenapparaturen sowie Abluftprobleme entstehen.

Ein weiterer Nachteil des in der DE-PS 937 646 beschriebenen Verfahrens besteht darin, daß für die Umsetzung 100%iges Pyren eingesetzt wird. Eine derartige Pyrenqualität ist aber wegen der außerordentlich hohen Herstellkosten für ein wirtschaftliches Verfahren nicht einsetzbar.

Außerdem wurde bei der Nacharbeitung festgestellt, daß die in der DE-PS 937 646 angegebene theoretische Ausbeute lediglich aus der Gewichtszunahme des Pyrens bei der Bromierung errechnet wurde und daß die wirkliche Ausbeute unter 90% der Theorie liegt. Die Ursache für diese Diskrepanz liegt darin, daß bromiertes Pyren im Gegensatz zu den Angaben der Literatur [Annalen 531 (1937), Seite 16] mit Brom ebenfalls Additionsverbindungen bilden kann und durch die dann eintretende Gewichtszunahme eine höhere Ausbeute an Tetrabrompyren vorgetäuscht wird.

Es wurde nun gefunden, daß man 1,3,6,8-Tetrabrompyren der vorstehend genannten Formel (1) in wäßrigem Medium unter Vermeidung der den genannten bekannten Verfahren anhaftenden Mängel und in höherer Ausbeute auf technisch einfache und ökologisch unbedenkliche Weise herstellen kann, indem man Pyren, insbesondere technisches Rohpyren, in hochkonzentrierter wäßriger Suspension mit einem Pyren-Wasser-Gewichtsverhältnis von 1 : 1,2 bis 2, vorzugsweise 1 : 1,5 bis 1,7 mit Brom bei einem Pyren-Brom-Gewichtsverhältnis von 1 : 1,6 bis 2 bei Temperaturen von 35° bis 65°C, vorzugsweise 45° bis 55°C, umsetzt bei gleichzeitiger Reoxidation des entstehenden Bromwasserstoffs zu Brom mit einem Oxidationsmittel, die Bromierung durch Erwärmen des Reaktionsgemisches auf Temperaturen von 70 bis 130°C, vorzugsweise 85 bis 100°C vervollständigt, überschüssiges Brom mit einem Reduktionsmittel reduziert zu Bromwasserstoff und das gebildete 1,3,6,8-Tetrabrompyren vorzugsweise ohne Zwischenisolierung einer alkalischen Nachbehandlung bei Gegenwart von Ammoniak bei Temperaturen von 40° bis 100°C im pH-Bereich 8,5 bis 12 unterwirft.

Geeignete Oxidationsmittel zur Reoxidation des bei der Bromierung des Pyrens mit Brom entstehenden Bromwasserstoffs sind beispielsweise die Alkalimetall- und Erdalkalimetall-hypochlorite, hypobromite, -peroxodisulfate, -chlorate und -bromate, vorzugsweise die Alkalimetallchlorate, wie das Natriumchlorat. Das bei der Reoxidation des entstandenen Bromwasserstoffs gebildete Brom geht unmittelbar wieder in den Bromierungsprozess ein, wodurch die Menge an einzusetzendem Brom wesentlich niedriger gehalten werden kann.

Geeignete Reduktionsmittel zur Reduktion von überschüssigem Brom zu Bromwasserstoff sind beispielsweise Schwefeldioxid sowie Salze der schwefligen Säure, d.h. Sulfite und Bisulfite, vorzugsweise die Alkalimetall- und Erdalkalimetallsulfite- und -bisulfite, insbesondere die Natriumsalze. Das gasförmige Schwefeldioxid ist insofern vorteilhaft, als es das in grobkörnigem Tetrabrompyren in Form von Einschlüssen enthaltene Brom zu Bromwasserstoff zu reduzieren vermag.

Als Pyren wird vorzugsweise das handelsübliche, bei der fraktionierten Destillation des Steinkohlenteers anfallende technische Rohpyren, das üblicherweise einen Reinheitsgehalt von 85 - 95% besitzt, verwendet. Dieses Rohpyren enthält eine Reihe von Verunreinigungen, die einen dem Pyren vergleichbaren Dampfdruck besitzen, von denen Brasan und Fluoranthen die Hauptkomponenten sind. Diese Verunreinigungen werden im Rahmen der Bromierung ebenfalls mehrfach bromiert. Brasan, das als Hauptverunreinigung im technischen Pyren in einer Menge von 2 - 10% vorkommt, wird z.B. tetra- bis pentabromiert.

Zur Bromierung in hochkonzentrierter wäßriger Suspension wird das technische Rohpyren üblicherweise zunächst in der 1,2- bis 2fachen, vorzugsweise 1,5- bis 1,7fachen Gewichtsmenge Wasser (bezogen auf eingesetztes Pyren) angerührt und anschließend bromiert. Die Wassermenge ist nach unten dadurch begrenzt, daß die Bromierungssuspension noch rührbar sein muß. Da mit der Bildung des Tetrabrompyrens eine erhebliche Gewichtszunahme verbunden ist, enthält die Bromierungssuspension

gegen Ende der Bromierung einen Feststoffgehalt von über 50%.

Die Brommenge für die Bromierung wird unter Berücksichtigung des durch Reoxidation gebildeten Broms so festgelegt, daß während der gesamten Bromierung ein Bromüberschuß vorliegt. Dabei ist aber darauf zu achten, daß gegen Ende der Umsetzung nur noch ein leichter Bromüberschuß vorliegt, weil es sonst unter Addition von 1 - 2 Mol Brom an das Tetrabrompyren zur Bildung von unerwünschten Bromadditionsverbindungen kommt. Die eingesetzte Brommenge ist außerdem abhängig von Art und Menge des zur Reoxidation des entstehenden Bromwasserstoffs zu Brom verwendeten Oxidationsmittels.

Für die erfindungsgemäße Bromierung wird die 1,6- bis 2fache, vorzugsweise die 1,7fache Gewichtsmenge an Brom, bezogen auf das eingesetzte Rohpyren, verwendet.

Weil das Tetrabrompyren nach vorausgegangener Behandlung mit einem der weiter oben genannten Reduktionsmittel zur Entfernung von überschüssigem Brom auch nach intensivem Waschen des durch Filtration isolierten Produkts beim Trocknen Bromwasserstoff freisetzt, wodurch Korrosions- und Abluftprobleme auftreten, ist die erfindungsgemäße alkalische Nachbehandlung des Tetrabrompyrens in der angefallenen Reaktionsmischung erforderlich. Hierzu wird vorzugsweise der pH-Wert der angefallenen Reaktionsmischung zunächst durch Zugabe eines Alkalimetallhydroxids, vorzugsweise Natriumhydroxid, zweckmäßigerweise in Form einer wäßrigen Lösung, auf 6 - 10 gestellt. Anschließend wird Ammoniak, gasförmig oder in Form einer wäßrigen Lösung, zugegeben und das Reaktionsgemisch der mehrstündigen Nachbehandlung bei Temperaturen von 40 bis 100°C, gegebenenfalls bei geschlossenem Gefäß, unterworfen. Das derart nachbehandelte Tetrabrompyren setzt beim Trocknen keine korrosive Verbindungen, wie Bromwasserstoff, frei.

Die alkalische Nachbehandlung des Tetrabrompyrens kann zwar auch in der Weise erfolgen, daß das Tetrabrompyren zunächst isoliert und dann in einer zweiten Stufe einer alkalischen Nachbehandlung unterworfen wird, doch ist der alkalischen Nachbehandlung der angefallenen Suspension (Reaktionsmischung) ohne Zwischenisolierung des Tetrabrompyrens der Vorzug zu geben.

Nachstehend seien noch einige Einzelheiten der Verfahrensdurchführung angegeben:

Bei Verwendung von Chloraten als Reoxidationsmittel wird die Zusatzmenge so festgelegt, daß am Ende der Bromierung das gesamte Chlorat verbraucht ist. Das Chlorat kann im Verlauf der Bromierung in kleinen Portionen oder kontinuierlich zugegeben werden, doch wird die Zugabe der gesamten Chloratmenge vor Bromierungsbeginn bevorzugt. Das Chlorat kann sowohl in fester Form als auch in Form einer konzentrierten wäßrigen Lösung zugesetzt werden. Bei Verwendung einer wäßrigen Chloratlösung muß die zum Anrühren des Pyrens verwendete Wassermenge entsprechend reduziert werden, um die erforderliche hohe Konzentration der Pyrensuspension zu gewährleisten. Im Hinblick auf die Temperaturführung wird die Bromierung in ihrer bevorzugten Form so durchgeführt, daß nach Zugabe des Natriumchlorats zur wäßrigen Pyrensuspension das Brom bei einer Temperatur von 35° bis 65°C, vorzugsweise bei 45° bis 55°C, unter leichter Kühlung langsam zuläuft und nach beendeter Zugabe noch einige Zeit in dem genannten Temperaturbereich nachgerührt wird. Bei dieser Reaktionstemperatur verläuft sowohl die Bromierung als auch die Reoxidation des entstehenden Bromwasserstoffs zu Brom relativ rasch. Im Gegensatz dazu hat die Bromierung bei niedrigerer Temperatur, insbesondere bei Verwendung von Chloraten als Oxidationsmittel den Nachteil, daß die Reoxidation des Bromwasserstoffs zu langsam verläuft und es beim anschließenden Erhitzen des Bromierungsgemisches zu einem Reaktionsstoß mit kräftigem Temperaturanstieg kommt. Ein Temperaturanstieg über 65°C ist aber insbesondere zu Beginn der Bromierung, unbedingt zu vermeiden, da es sonst zu irreversibler Klumpenbildung kommt, die eine erfolgreiche Bromierung verhindert. Zur Vervollständigung der Bromierung wird das Reaktionsgemisch anschliessend mehrere Stunden bei 70° bis 130°C erwärmt. Dies kann zwar in geschlossenem Gefäß unter Druck erfolgen, doch wird die Vervollständigung der Bromierung unter Normaldruck durch Kochen am Rückfluß bevorzugt.

Nach beendeter Bromierung wird die Bromierungssuspension, die aufgrund des Gehalts an Bromwasserstoffsäure stark sauer ist, vorzugsweise mit Wasser verdünnt und das überschüssige Brom, gegebenenfalls nach vorherigem Abdestillieren der Hauptmenge des Broms, durch Zugabe eines Reduktionsmittels zu Bromwasserstoff reduziert.

Der Vorteil des beanspruchten Verfahrens gegenüber dem Verfahren des DE-PS 937 646 besteht in folgenden Punkten:

1) Man erhält eine wesentlich bessere Ausbeute an 1,3,6,8-Tetrabrompyren; außerdem wird eine sehr hohe Raum-Zeit-Ausbeute erzielt;
2) aufgrund der konzentrierten Arbeitsweise kann die technisch sehr aufwendige Mahlung des Pyrens vor Beginn der Bromierung entfallen;
3) das bei der Filtration des Tetrabrompyrens anfallende Abwasser ist nicht verunreinigt durch Tenside und Schwefelsäure;
4) Korrosionsprobleme bei der Trocknung des Tetrabrompyrens entfallen;
5) ausgehend von dem verfahrensgemäss hergestellten Tetrabrompyren wird, bezogen auf den Pyreneinsatz, bei der Weiterverarbeitung zur Naphthalin-1,4,5,8-tetracarbonsäure eine um mindestens 5% höhere Ausbeute an dieser Verbindung erzielt als bei Tetrabrompyren, das gemäß der DE-PS 937 646 hergestellt wurde.

Das erfindungsgemäße Verfahren ist neu. Es war nicht voraussehbar, dass bei Bromierung in hochkonzentrierter wäßriger Pyrensuspension ohne jeden vorherigen Feinverteilungsaufwand eine höhere Ausbeute an 1,3,6,8-Tetrabrompyren als nach dem in der DE-PS 937 646 beschriebenen Verfahren erzielt wird.

Das nach dem beanspruchten Verfahren erhaltene 1,3,6,8-Tetrabrompyren findet Verwendung als organisches Zwischenprodukt, insbesondere zur Herstellung von Naphthalin-1,4,5,8-tetracarbonsäure bzw. dessen Monoanhydrid.

Die in den Beispielen genannten Teile und Prozente sind Gewichtsteile und Gewichtsprozente.

*Beispiel 1*

a) 220 Teile technisches Pyren von 90%iger Reinheit (Gehalt an Brasan ca. 6%) werden in eine Lösung von 84 Teilen Natriumchlorat in 360 Teilen Wasser eingerührt. Dann lässt man unter leichter Kühlung im Verlauf von 1$^1/_2$ bis 2 Stunden bei 45° - 55°C 371 Teile Brom zutropfen. Man rührt 1 bis 2 Stunden bei 45° - 55°C nach. Anschließend erwärmt man langsam unter Rückflußkühlung auf 85° - 95°C und rührt 8 Stunden bei 85° - 100°C nach. Der anfangs noch kräftige Bromrückfluß verschwindet im Laufe der Umsetzung fast völlig. Nach beendeter Umsetzung verdünnt man die Suspension mit 280 Teilen Wasser, wobei man die Temperatur auf 60° - 70°C absinken läßt. Dann entfernt man den Bromüberschuß durch langsame Zugabe von etwa 30 Teilen einer 40%igen Natriumsulfitlösung. Anschliessend stellt man die Suspension durch Zugabe von etwa 25 Teilen einer 33%igen Natronlauge auf pH 6,5 - 7,5. Darauf gibt man 40 Teile einer wäßrigen 25%igen Ammoniaklösung zu, wobei sich ein pH-Wert von 9 - 10 einstellt. Man rührt 4 Stunden bei 60° - 70°C nach. Schließlich wird abgesaugt, mit Wasser salzfrei gewaschen und bei 100°C getrocknet. Das Produkt gibt beim Trocknen keine Bromwasserstoffsäure ab.

Man erhält 558 Teile 1,3,6,8-Tetrabrompyren (Bromgehalt: 61,7%) eines Reinheitsgrades von etwa 83%. Dies entspricht, bezogen auf 100%iges Pyren, einer Ausbeute von ca. 91% der Theorie. Bei der Weiterverarbeitung dieses Tetrabrompyrens zur Naphthalin-1,4,5,8-tetracarbonsäure durch Verseifung in Oleum und anschließende Hypochloritoxidation des isolierten Zwischenproduktes erhält man 252 Teile Naphthalin-1,4,5,8-tetracarbonsäure-1,8-monoanhydrid, entsprechend einer Ausbeute von 89,9% der Theorie, bezogen auf 100%iges Pyren.

Die Reinheitsbestimmung des technischen Tetrabrompyrens erfolgt unter Ausnutzung der Tatsache, daß die Verunreinigungen im Gegensatz zu 1,3,6,8-Tetrabrompyren in o-Dichlorbenzol sehr leicht löslich sind, nach folgender Methode:

50 Teile des nach Beispiel 1a erhaltenen Tetrabrompyrens werden in feingemahlener Form in 1000 Teile o-Dichlorbenzol eingetragen und 2 Stunden bei 160° - 170°C gerührt. Dann kühlt man auf 60° - 70°C ab und saugt bei dieser Temperatur ab. Man wäscht den Filterkuchen mit 500 Teilen o-Dichlorbenzol nach. Anschließend wäscht man den Filterkuchen mit Methanol frei von o-Dichlorbenzol und trocknet bei 100°C. Man erhält 41,4 Teile reines Tetrabrompyren.

b) Verwendet man bei der Bromierung gemäß Beispiel 1a anstelle von 371 Teilen Brom eine Brommenge von nur 340 Teilen, so erhält man nur 540 Teile 1,3,6,8-Tetrabrompyren eines Reinheitsgrades von nur ca. 74%. Dies entspricht, bezogen auf 100%iges Pyren, einer Ausbeute von ca. 79% der Theorie.

c) Verwendet man bei der Bromierung gemäß Beispiel 1a anstelle von 371 Teilen Brom eine Brommenge von 420 Teilen, so liegt auch gegen Ende der Bromierung noch ein sehr starker Bromüberschuß vor. Das Tetrabrompyren fällt in einer äußerst grobkörnigen und spezifisch sehr schweren Form an, die technisch nur sehr schwer zu handhaben ist. Man erhält in diesem Fall infolge der Addition von Brom an das Tetrabrompyren zwar eine Ausbeute von 578 Teilen «Tetrabrompyren» (Bromgehalt 64 - 65%), die aber bei der Weiterverarbeitung nur 243 Teile Naphthalin-1,4,5,8-tetracarbonsäure-1,8-monoanhydrid ergeben.

d) Wird vor der Isolierung des Tetrabrompyrens auf die Ammoniaknachbehandlung verzichtet und das Tetrabrompyren aus der neutralen Suspension durch Filtration isoliert und gründlich mit Wasser gewaschen, so wird bei der Trocknung noch Bromwasserstoff freigesetzt, der eine erhebliche Abluftbelastung darstellt und zur Korrosion der Trockenapparatur führt.

*Beispiel 2*

(Nacharbeitung von Beispiel 2 der DE-PS 937 646)

220 Teile technisches Pyren von 90%iger Reinheit (identisch mit dem in Beispiel 1 verwendeten Pyren) werden mit 6,6 Teilen dibutylnaphthalinsulfonsaurem Natrium und 440 Teilen Wasser 96 Stunden in einer Rollmühle gemahlen. Die Suspension wird mit 880 Teilen Wasser in einen Kolben gespült. Dann werden in 2 Stunden bei 20° - 30°C 374 Teile Brom zugetropft. Nach Verschwinden der Bromfarbe werden 74,8 Teile 100%ige Schwefelsäure zugesetzt. Nach Erwärmen auf 60°C wird bei 60° - 70°C in 5 Stunden eine Lösung von 79,2 Teilen Natriumchlorat in 330 Teilen Wasser zugetropft. Anschließend wird auf 85° bis 90°C erhitzt und 11 Stunden bei 85°-90°C gehalten. Nach dem Abkühlen wird abgesaugt, mit Wasser neutral gewaschen und bei 100°C getrocknet.

Bei der Trocknung wird Bromwasserstoff freigesetzt. Man erhält 544 Teile 1,3,6,8-Tetrabrompyren von nur 73%iger Reinheit. Dies entspricht, bezogen auf 100%iges Pyren, einer Ausbeute von ca. 78% der Theorie.

*Beispiel 3*

a) 220 Teile technisches Pyren 93%iger Reinheit (Brasangehalt 4%) werden in eine Lösung von 84 Teilen Natriumchlorat in 340 Teilen Wasser eingetragen. Dann lässt man unter leichter Kühlung in 2 Stunden bei 40° - 50°C 375 Teile Brom zutropfen. Man rührt 1 - 2 Stunden bei 40° - 50°C nach. Anschließend erwärmt man langsam unter Rückflußkühlung auf 85°C und rührt 5 Stunden bei 85° - 95°C nach. Darauf verdünnt man die Suspension mit 200 Teilen Wasser und beseitigt den Bromüberschuß durch langsame Zugabe von 40%iger wäßriger Natriumbisulfitlösung. Dann kühlt man auf 50° - 60°C ab und tropft 25%ige wässrige Ammoniaklösung zu, bis ein pH-Wert von 9,5 erreicht ist. Man rührt 4 Stunden bei 50° - 60°C nach. Schließlich wird abgesaugt, mit Wasser salzfrei gewaschen und bei 100°C getrocknet.

Man erhält 561 Teile 1,3,6,8-Tetrabrompyren eines Reinheitsgrades von ca. 84%, was einer Ausbeute von ca. 90% der Theorie, bezogen auf 100%iges Pyren, entspricht, die bei der Weiterverarbeitung zur Naphthalin-1,4,5,8-tetracarbonsäure gemäß den Ausführungen von Beispiel 1a 263 Teile Naphthalin-1,4,5,8-tetracarbonsäure-1,8-monoanhydrid ergeben.

b) Wird das Brom nicht bei 40° - 50°C sondern bei 20° - 30°C zugetropft, so tritt beim anschließenden Erwärmen beim Erreichen von etwa 40°C eine sehr heftige exotherme Reaktion ein, die einen raschen Temperaturanstieg zur Folge hat und die beim Arbeiten in technischer Größenordnung zum Durchgehen der Temperatur und damit zu einem außerordentlichen Sicherheitsrisiko infolge von Bromausbrüchen führen kann.

c) Zur Reduktion des Bromüberschusses nach beendeter Bromierung kann anstelle einer Natriumbisulfitlösung mit gleichem Erfolg auch gasförmiges Schwefeldioxyd verwendet werden.

d) Ein Tetrabrompyren, das bei der Trocknung keinen Bromwasserstoff freisetzt, wird auch erhalten, wenn das Tetrabrompyren nach der Reduktion des Broms durch Filtration isoliert wird, nach dem Neutralwaschen in 1000 Teile 3%igen wäßrigen Ammoniak eingetragen und 2 Stunden bei 60° - 70°C gerührt wird und anschließend erneut abgesaugt, mit Wasser gewaschen und getrocknet wird.

*Beispiel 4*

a) 220 Teile technisches Pyren von 90%iger Reinheit werden in eine Lösung von 84 Teilen Natriumchlorat und 350 Teilen Wasser eingetragen.

Dann läßt man in 2 Stunden bei 60° - 65°C 370 Teile Brom zutropfen. Man rührt 1 Stunde bei 60° - 65°C nach. Anschließend erwärmt man auf 85°C und rührt unter Rückflußkühlung 10 Stunden bei 85° - 95°C. Die Aufarbeitung des Tetrabrompyrens erfolgt wie in Beispiel 1a beschrieben.

Man erählt 557 Teile 1,3,6,8-Tetrabrompyren (Bromgehalt 61,8%) eines Reinheitsgrades von etwa 83%, was einer Ausbeute von ca. 91% der Theorie, bezogen auf 100%iges Pyren, entspricht, die bei der Weiterverarbeitung 253 Teile Naphthalin-1,4,5,8-tetracarbonsäure-1,8-monoanhydrid liefern.

b) Lässt man bei der Bromzugabe gemäß Beispiel 4a das Brom nicht bei 60° - 65°C sondern bei 70° - 75°C zutropfen, dann beginnt ein Teil des Reaktionsproduktes schon kurze Zeit nach Beginn der Bromzugabe zu schmelzen und es bildet sich eine schmierige Masse, die in dieser Form nicht mehr bromierbar ist.

Erfolgt die Bromzugabe dagegen zunächst bei 60° - 65°C und wird erst nach Zugabe der Hauptmenge Brom auf 70° - 75°C weiter erhitzt, dann läßt sich die Bromierung problemlos durchführen.

c) Verwendet man bei der Bromierung gemäß Beispiel 4a 90 Teile Natriumchlorat anstelle von 84 Teilen, dann erhält man Tetrabrompyren von gleicher Qualität und Ausbeute, wenn man die Brommenge auf 360 Teile reduziert.

*Beispiel 5*

220 Teile technisches Pyren von 90%iger Reinheit werden in 240 Teile Wasser eingetragen. Dann läßt man bei 35° - 40°C in 2 Stunden 370 Teile Brom zutropfen. Man rührt 1 Stunde bei 35° - 40°C nach. Anschließend läßt man bei 45° - 55°C in 1 - 2 Stunden eine Lösung von 84 Teilen Natriumchlorat in 100 Teilen Wasser zutropfen. Man rührt 1 Stunde bei 45 - 55°C nach. Dann erwärmt man auf 85°C und rührt 6 Stunden bei 85° - 95°C.

Die Aufarbeitung des Tetrabrompyrens erfolgt wie in Beispiel 1a beschrieben, wobei aber die Suspension nach der Ammoniakbehandlung und vor der Isolierung des Tetrabrompyrens durch Zugabe von Salzsäure auf pH 7 gestellt wird.

Man erhält 556 Teile 1,3,6,8-Tetrabrompyren (Bromgehalt 61,7%) eines Reinheitsgrades von etwa 83%, was einer Ausbeute von ca. 91% der Theorie, bezogen auf 100%iges Pyren, entspricht, die bei der Weiterverarbeitung gemäss Beispiel 1a 252 Teile Naphthalin-1,4,5,8-tetracarbonsäure-1,8-monoanhydrid ergeben.

*Beispiel 6*

220 Teile technisches Pyren von 90%iger Reinheit werden in eine Lösung von 84 Teilen Natriumchlorat in 440 Teilen Wasser eingetragen. Dann läßt man in 2 Stunden bei 40° - 50°C 380 Teile Brom zutropfen. Man rührt 1 Stunde bei 40° - 50°C nach. Anschließend verschließt man das Reaktionsgefäß und erwärmt langsam unter Druck auf 120°C. Man rührt 8 Stunden unter Druck bei 120° - 130°C. Darauf kühlt man auf 60° - 70°C ab und arbeitet das Tetrabrompyren, wie in Beispiel 1a beschrieben, auf.

Man erhält 556 Teile 1,3,6,8-Tetrabrompyren eines Reinheitsgrades von ca. 83%, was einer Ausbeute von ca. 91% der Theorie, bezogen auf 100%iges Pyren, entspricht.

*Beispiel 7*

a) 220 Teile technisches Pyren von 90%iger Reinheit werden in eine Lösung von 84 Teilen Natriumchlorat in 280 Teilen Wasser eingetragen. Dann läßt man unter leichter Kühlung in 2 Stunden bei 40° - 50°C Teile Brom zutropfen. Man rührt 1 Stunde bei 40° - 50°C nach. Anschließend erwärmt man langsam unter Rückflußkühlung auf 85°C und rührt 10 Stunden bei 85° - 95°C nach. Die Aufarbeitung der Suspension erfolgt wie in Beispiel 1a beschrieben.

Man erhält 564 Teile 1,3,6,8-Tetrabrompyren von etwa 83%iger Reinheit, was einer Ausbeute von ca. 92% der Theorie, bezogen auf 100%iges Pyren, entspricht, die bei der Weiterverarbeitung zur Naphthalin-1,4,5,8-tetracarbonsäure 258 Teile Naphthalin-1,4,5,8-tetracarbonsäure-1,8-monoanhydrid ergeben.

b) Führt man die Bromierung gemäß Beispiel 7a in stärkerer wäßriger Verdünnung durch und verwendet 2000 Teile Wasser anstelle von 280 Teilen, so erhält man nur eine Ausbeute von 535 Teilen 1,3,6,8-Tetrabrompyren, die bei der Weiterverarbeitung gemäß Beispiel 1a nur 238 Teile Naphthalin-1,4,5,8-tetracarbonsäure-1,8-monoanhydrid ergeben.

Ein ähnlich ungünstiges Resultat erhält man, wenn man anstelle von 2000 Teilen Wasser 900 Teile Wasser einsetzt.

**Patentansprüche**

1. Verfahren zur Herstellung von 1,3,6,8-Tetrabrompyren durch Bromierung von Pyren in wäßriger Suspension unter gleichzeitiger Reoxidation des entstehenden Bromwasserstoffs zu Brom mittels eines Oxidationsmittels, dadurch gekennzeichnet, daß man Pyren in hochkonzentrierter wäßriger Suspension mit einem Pyren-Wasser-Gewichtsverhältnis von 1 : 1,2 bis 2 mit Brom mit einem Pyren-Brom-Gewichtsverhältnis von 1 : 1,6 bis 2 bei Temperaturen von 35° bis 65°C umsetzt bei gleichzeitiger Reoxidation des entstehenden Bromwasserstoffs zu Brom mit einem Oxidationsmittel, die Bromierung durch Erwärmen des Reaktionsgemisches auf Temperaturen von 70 bis 130°C vervollständigt, überschüssiges Brom mit einem Reduktionsmittel reduziert zu Bromwasserstoff und das gebildete 1,3,6,8-Tetrabrompyren, vorzugsweise ohne Zwischenisolierung, einer alkalischen Nachbehandlung bei Gegenwart von Ammoniak bei Temperaturen von 40° bis 100°C im pH-Bereich 8,5 bis 12 unterwirft.

2. Verfahren nach Anspruch 1, dadurch gekennzeichnet, daß man als Pyren technisches Rohpyren von 85 bis 95%iger Reinheit einsetzt.

3. Verfahren nach mindestens einem der Ansprüche 1 und 2, dadurch gekennzeichnet, daß das Pyren-Wasser-Gewichtsverhältnis 1 : 1,5 bis 1,7 beträgt.

4. Verfahren nach mindestens einem der Ansprüche 1 bis 3, dadurch gekennzeichnet, daß man die Bromierung bei Temperaturen von 45° bis 55°C durchführt.

5. Verfahren nach mindestens einem der Ansprüche 1 bis 4, dadurch gekennzeichnet, daß das Pyren-Brom-Gewichtsverhältnis 1 : 1,7 beträgt.

6. Verfahren nach mindestens einem der Ansprüche 1 bis 5, dadurch gekennzeichnet, daß man zur Reoxidation des entstehenden Bromwasserstoffs zu Brom die Alkalimetall- oder Erdalkalimetallhypochlorite, -hypobromite, -peroxodisulfate, -chlorate oder -bromate verwendet.

7. Verfahren nach Anspruch 6, dadurch gekennzeichnet, daß man als Reoxidationsmittel Natriumchlorat verwendet.

8. Verfahren nach mindestens einem der Ansprüche 1 bis 7, dadurch gekennzeichnet, daß man zur alkalischen Nachbehandlung den pH-Wert des Reaktionsgemisches zunächst durch Zugabe eines Alkalimetallhydroxids auf 6 - 10 stellt und anschließend Ammoniak gasförmig oder in Form einer wäßrigen Lösung zugibt.

## Claims

1. A process for preparing 1,3,6,8-tetrabromopyrene by brominating pyrene in aqueous suspension with simultaneous reoxidation of the resulting hydrogen bromide to bromine by means of an oxidizing agent, which comprises reacting pyrene, in a highly concentrated aqueous suspension having a pyrene-water weight ratio of 1 : 1.2 to 2, with bromine in a pyrene-bromine weight ratio of 1 : 1.6 to 2 at temperatures of 35° to 65°C with simultaneous reoxidation of the resulting hydrogen bromide to bromine using an oxidizing agent, completing the bromination by heating the reaction mixture to temperatures of 70 to 130°C, reducing excess bromine with a reducing agent to hydrogen bromide and subjecting the 1,3,6,8-tetrabromopyrene formed, preferably without intermediate isolation, to an alkaline aftertreatment in the presence of ammonia at temperatures of 40° to 100°C within the pH range 8.5 to 12.

2. The process as claimed in claim 1, wherein the pyrene used is technical-grade crude pyrene of 85 to 95% purity.

3. The process as claimed in at least one of claims 1 and 2, wherein the pyrene-water weight ratio is 1 : 1.5 to 1.7.

4. The process as claimed in at least one of claims 1 to 3, wherein the bromination is carried out at temperatures of 45° to 55°C.

5. The process as claimed in at least one of claims 1 to 4, wherein the pyrene-bromine weight ratio is 1 : 1.7.

6. The process as claimed in at least one of claims 1 to 5, wherein the resulting hydrogen bromide is reoxidized to bromine by means of alkali metal or alkaline earth metal hypochlorites, hypobromites, peroxodisulfates, chlorates or bromates.

7. The process as claimed in claim 6, wherein the reoxidizing agent used is sodium chlorate.

8. The process as claimed in at least one of claims 1 to 7, wherein, to carry out the alkaline aftertreatment, the pH value of the reaction mixture is first brought to 6 - 10 by adding alkali metal hydroxide and ammonia is then added in gas form or in the form of an aqueous solution.

## Revendications

1. Procédé pour préparer le tétrabromo-1,3,6,8 pyrène par bromation du pyrène en suspension aqueuse avec réoxydation simultanée, au moyen d'un agent d'oxydation, du bromure d'hydrogène formé pour le convertir en brome, procédé caractérisé en ce qu'on fait réagir le pyrène en suspension du pyrène à l'eau de 1 : 1,2 à 2, avec le brome, selon un rapport pondéral du pyrène au brome de 1 : 1,6 à 2, à des températures de 35 à 65°C, en même temps qu'on réoxyde en brome, au moyen d'un agent d'oxydation, le bromure d'hydrogène qui se forme, on complète la bromation en chauffant le mélange réactionnel à des températures de 70 à 130°C, on réduit l'excès de brome par un réducteur de manière à le convertir en bromure d'hydrogène, et on soumet le tétrabromo-1,3,6,8 pyrène formé, de préférence sans l'isoler intermédiairement, à un traitement complémentaire alcalin en présence d'ammoniac, à des températures de 40 à 100°C, dans l'intervalle de ph allant de 8,5 à 12.

2. Procédé selon la revendication 1, caractérisé en ce qu'on utilise, comme pyrène, du pyrène brut d'une pureté de 85 à 95%.

3. Procédé selon au moins une des revendications 1 et 2, caractérisé en ce que le rapport pondéral du pyrène à l'eau est de 1 : 1,5 à 1,7.

4. Procédé selon au moins une des revendications 1 à 3, caractérisé en ce qu'on effectue la bromation à des températures de 45 à 55°C.

5. Procédé selon au moins une des revendications 1 à 4, caractérisé en ce que le rapport pondéral du pyrène au brome est de 1 : 1,7.

6. Procédé selon au moins une des revendications 1 à 5, caractérisé en ce que, pour réoxyder le bromure d'hydrogène formé et le convertir ainsi en brome, on utilise des hypochlorites, des hypobromites, des peroxodisulfates, des chlorates ou des bromates de métaux alcalins ou de métaux alcalino-terreux.

7. Procédé selon la revendication 6, caractérisé en ce qu'on utilise le chlorate de sodium comme agent de réoxydation.

8. Procédé selon au moins une des revendications 1 à 7, caractérisé en ce que, pour le traitement complémentaire alcalin, on règle d'abord le pH du mélange réactionnel à une valeur de 6 à 10 par addition d'un hydroxyde de métal alcalin, puis on ajoute de l'ammoniac à l'état gazeux ou sous la forme d'une solution aqueuse.